# EUROPÄISCHE PATENTSCHRIFT

(11) **EP 0 893 094 B1**
(45) Veröffentlichungstag und Bekanntmachung des Hinweises auf die Patenterteilung: **05.11.2003**
(21) Anmeldenummer: 98113840.7
(22) Anmeldetag: 23.07.1998
(51) Int. Cl.: A61B 5/15

(54) **Blutentnahmevorrichtung**
Blood sampling device
Dispositif de prélèvement d'échantillons de sang

(30) Priorität: 23.07.1997 DE 19731744
(43) Veröffentlichungstag der Anmeldung: 27.01.1999
(73) Patentinhaber: Smiths Medical Deutschland GmbH, 85614 Kirchseeon (DE)
(72) Erfinder: Hermann Glowka, D-83367 Grafing (DE)
(74) Vertreter: von Bülow, Tam, Dr.

(56) Entgegenhaltungen:
- WO-A-91/01769
- US-A- 3 834 372
- US-A- 5 135 492
- Firmenprospekt der Firma PVB Medizintechnik mit dem Titel "HaemoGuard, the closest arterial blood sampling system for use during invasive blood monitoring" (November 1993).

## Beschreibung

Die Erfindung bezieht sich auf eine Blutentnahmevorrichtung mit einem Entnahmeport und einem Hahn gemäß dem Oberbegriff des Patentanspruches 1.

Eine derartige Blutentnahmevorrichtung ist aus dem Firmenprospekt der Firma pvb medizintechnik mit dem Titel "Haemo-Guard, the closest arterial blood sampling system for use during invasive blood pressure monitoring" (November 1993) bekannt.

In dieser Druckschrift ist ein Absperrhahn gezeigt, der über eine flexible Leitung mit einer Blutentnahmestelle verbunden ist, die im folgenden als "Entnahmeport" bezeichnet wird. Dieser Entnahmeport ist in einem Abstand vom Hahn angeordnet, ohne daß eine bestimmte Position relativ zum Hahn festgelegt ist.

Der Entnahmeport hat einen Hohlraum, in den von gegenüberliegenden Seiten je ein Kanal einmündet. Diese Kanäle sind mit je einer Leitung zum Leiten von Blut und/oder einer Infusionsflüssigkeit verbunden. Ferner weist der Hohlraum des Entnahmeports eine Öffnung auf, die durch einen Verschlußstopfen flüssigkeitsdicht verschlossen ist. Der Verschlußstopfen ist aus einem elastischen Werkstoff hergestellt und kann mit einer Blutentnahmenadel durchstochen werden, um eine im Hohlraum des Entnahmeports enthaltene Flüssigkeit, wie z.B. Blut, zu entnehmen.

Der Entnahmeport ist üblicherweise in einem Fluidsystem angeordnet, das Einrichtungen zur Infusion von Medikamenten und/oder zur arteriellen oder intravenösen Blutdruckmessung aufweist. Beispielsweise ist ein solches System wie folgt aufgebaut:

Ausgehend von einer in ein Blutgefäß des Patienten eingeführten Nadel führt ein Schlauch zum Entnahmeport, von dort über einen ersten Absperrhahn zu einem Reservoir und von dort über einen zweiten Absperrhahn zu einem Druckmeßfühler und von dort zu einem Behälter für Spülflüssigkeit, wie z.B. physiologischer Kochsalzlösung, deren Zweck es ist, das Leitungssystem freizuhalten und Blutkoagulationen im Leitungssystem zu verhindern. Während der Blutdruckmessung sind beide genannten Absperrhähne geöffnet. Am Entnahmeport befindet sich je nach Schlauchlänge in diesem Zustand reine Spülflüssigkeit oder mit Spülflüssigkeit verdünntes Blut. Soll nun für Analysezwecke am Entnahmeport Blut entnommen werden, so muß vorher dafür Sorge getragen werden, daß dort reines, unverdünntes Blut vorhanden ist. Hierzu wird zunächst der zweite Hahn abgesperrt, um ein Nachspeisen von Spülflüssigkeit zu unterbinden. Der erste Absperrhahn ist noch geöffnet. Nun wird das Reservoir gefüllt, so daß Spülflüssigkeit und verdünntes Blut aus dem Leitungssystem soweit zurückgezogen werden, daß am Entnahmeport nur noch reines Blut vorhanden ist. Anschließend wird der erste Absperrhahn geschlossen und am Entnahmeport kann reines Blut entnommen werden. Nach der Entnahme wird der erste Absperrhahn wieder geöffnet und der Inhalt des Reservoirs wird in das System zurück zum Patienten gedrückt. Abschließend wird dann auch der zweite Hahn wieder geöffnet.

Die korrekte Bedienung des Systems erfordert eine gründliche Schulung des medizinischen Personals und auch eine hohe Aufmerksamkeit des Personals, die in Streßsituationen nicht immer gegeben ist. So kann es beispielsweise vorkommen, daß am Entnahmeport noch verdünntes Blut vorliegt und somit eine für Analysezwecke unbrauchbare Blutprobe entnommen wird. Eine andere Fehlbedienung kann darin liegen, daß während des Füllens des Reservoirs der erste und der zweite Hahn geschlossen sind. Dadurch wird im Reservoir ein Unterdruck erzeugt, der beim anschließenden Öffnen des zweiten Hahns zu einer Gasblasenbildung führen kann.

Aufgabe der Erfindung ist es daher, eine Blutentnahmevorrichtung zu schaffen, die sicherstellt, daß eine Blutentnahme am Entnahmeport nur bei ordnungsgemäßer Stellung des mit dem Entnahmeport verbundenen Hahns möglich ist und die kostengünstig herstellbar ist.

Diese Aufgabe wird durch die im Patentanspruch 1 angegebenen Merkmale gelöst. Vorteilhafte Ausgestaltungen und Weiterbildungen der Erfindung sind den Unteransprüchen zu entnehmen.

Das Grundprinzip der Erfindung besteht darin, einen Entnahmeport und einen Hahn derart aneinander zu koppeln, daß in Abhängigkeit von der Stellung des Hahns der Verschlußstopfen des Entnahmeports abgedeckt oder zur Blutabnahme freigegeben ist.

Dies hat den großen Vorteil, daß eine Blutentnahme aus dem Entnahmeport nur in einer oder mehreren bestimmten Stellungen des Hahns möglich und ansonsten blockiert ist. So ist es sinnvoll, daß ein Zugang zum Verschlußstopfen des Entnahmeports nur dann möglich ist, wenn der Hahn geschlossen ist, wodurch eine Vermischung von abzunehmenden reinem Blut mit Infusionsflüssigkeit verhindert wird. Entsprechend ist beim Zurückziehen von Flüssigkeit durch das Reservoir eine Blutentnahme am Entnahmeport ausgeschlossen. Eine Fehlbedienung einer derartigen Entnahmeport-Hahn-Kombination ist unmöglich.

Realisierbar ist eine derartige Blutentnahmevorrichtung dadurch, daß der Entnahmeport relativ zum Hahn raumfest angeordnet ist und der Verschlußstopfen des Entnahmeports durch ein bewegliches Teil des Hahns abdeckbar ist. Das bewegliche Teil des Hahns kann mit dem Küken gekoppelt sein, wenn das Gehäuse des Hahns relativ zum Entnahmeport raumfest ist; alternativ ist es auch möglich, das Küken raumfest zum Entnahmeport anzuordnen, so daß das Gehäuse beweglich ist und das bewegliche abdeckende Teil mit dem Gehäuse gekoppelt ist. Somit ist in Abhängigkeit von der Stellung des Hahns der Verschlußstopfen des Entnahmeports durch das entsprechende bewegliche Teil des Hahns blockiert oder zugänglich. Eine derartige "mechanische Kopplung" von Hahn und Entnahmeport ist kostengünstig zu realisieren und ausfallssicher.

Vorzugsweise ist das bewegliche Teil des Hahns, das den Verschlußstopfen des Entnahmeports abdeckt bzw. freigibt, ein Griff oder ein Teil des Griffes des Hahns.

Nach einer Weiterbildung der Erfindung ist das Griffstück des Hahns ein Betätigungshebel oder ein plattenartiges Betätigungselement mit mindestens einer Öffnung. Ein Betätigungsgriff kann beispielsweise einen oder mehrere Hebelarme aufweisen, deren Breite in azimutaler Richtung zur Drehachse des Hahns individuell gestaltbar ist und die somit in einer oder mehreren Hahnstellungen den Verschluß des Entnahmeports abdecken bzw. freigeben. Alternativ zu einem einen Hebel aufweisenden Griff kann auch ein plattenartiges Griffelement mit einer oder mehreren lochartigen Öffnungen verwendet werden. Je nach Stellung des Hahns deckt das plattenartige Griffelement entweder den Verschluß des Entnahmeports ab oder die mindestens eine Öffnung des Griffelements liegt über dem Verschluß, so daß dieser mit einer Nadel zugänglich ist. Beide "Griffelementtypen" sind kostengünstig herstellbar.

Nach einer Weiterbildung der Erfindung sind der Entnahmeport und der Hahn in ein gemeinsames Gehäuse integriert. Da es üblich ist, Gehäuse für Blutentnahmeports und medizinische Hähnchen im Spritzgußverfahren aus Kunststoff herzustellen, läßt sich durch eine Kombination zweier solcher Gehäuse ein kompletter Arbeitsgang und somit Kosten einsparen. Ferner ist durch Verwendung eines gemeinsamen Gehäuses für den Entnahmeport und den Hahn die Baugröße der Blutentnahmevorrichtung minimierbar.

Nach einer Weiterbildung der Erfindung ist der Entnahmeport und der Hahn auf einem als Grundplatte fungierenden Bauelement angeordnet. Somit ist es auch möglich, den Entnahmeport und den Hahn in Form separater Bauteile raumfest zueinander anzuordnen. Hierfür können sogar manche handelsübliche Entnahmeports und Hähne verwendet werden, ohne daß eigens ein Entnahmeport und ein Hahn konstruiert werden müssen. Sehr kostengünstig lassen sich der Entnahmeport bzw. der Hahn mit der Grundplatte über einen Schnappverschluß oder über Halteschienen verbinden. Dadurch ist eine rationelle Montage und ggf. auch eine schnelle Demontage möglich.

Nach einer Weiterbildung der Erfindung sind der Entnahmeport und der Hahn unmittelbar aneinander koppelbar und somit ebenfalls raumfest zueinander ausgerichtet. Somit ist es auch möglich, einen Entnahmeport und einen Hahn in Form separater Bauteile ohne Verwendung einer Grundplatte aneinander zu koppeln. Dies ist beispielsweise durch eine einfach zu montierende und kostengünstige Luerverbindung möglich.

Nach einer Weiterbildung der Erfindung ist die Grundplatte an einem Patienten befestigbar. Beispielsweise läßt sich die Grundplatte durch Gurte am Arm des Patienten festbinden, so daß die Leitungslänge zwischen einer in eine Ader oder Vene an der Hand des Patienten eingesteckten Nadel und der Blutentnahmevorrichtung minimierbar ist.

Im folgenden wird die Erfindung anhand von Ausführungsbeispielen im Zusammenhang mit der Zeichnung näher erläutert. Es zeigt:
- Fig. 1: einen Längsschnitt durch eine Blutentnahmevorrichtung gemäß der Erfindung bei geöffnetem Hahn;
- Fig. 2: den Längsschnitt durch die Blutentnahmevorrichtung der Fig. 1 bei geschlossenem Hahn;
- Fig. 3: eine Draufsicht auf einen Teilschnitt durch die Blutentnahmevorrichtung entlang der in Fig. 2 angegebenen Schnittlinie A - B;
- Fig. 4: eine Draufsicht auf eine Blutentnahmevorrichtung nach einem weiteren Ausführungsbeispiel ähnlich dem der Fig. 1 bis 3 mit kreisförmigem Griffstück; und
- Fig. 5: eine Blutentnahmevorrichtung mit einem Hahn, der als separates Bauteil mit dem Entnahmeport verbunden ist.

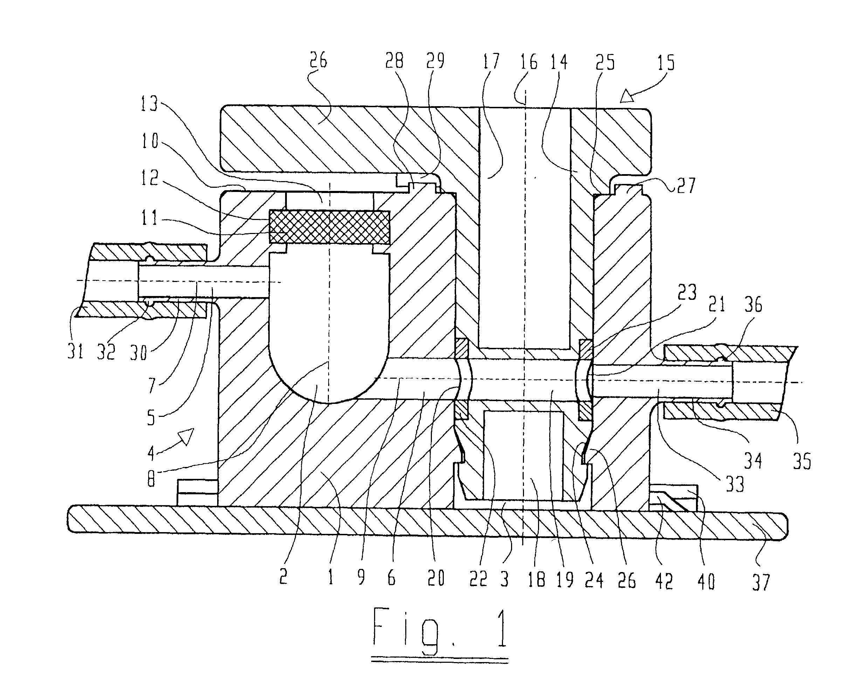

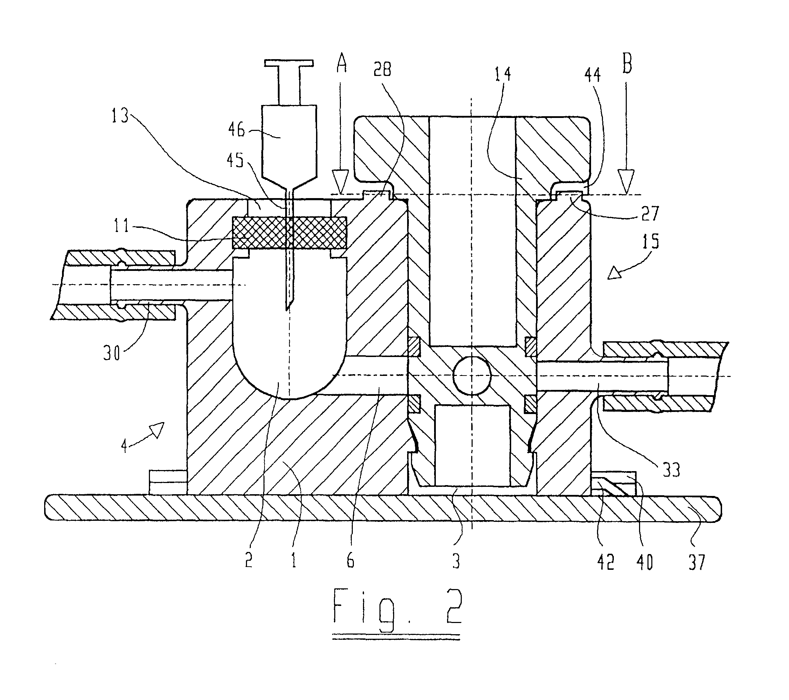

Fig. 1 zeigt eine Blutentnahmevorrichtung mit einem Gehäuse 1, in dem sich ein erster Hohlraum 2 und ein zweiter Hohlraum 3 befinden. Der Hohlraum 2 ist Teil eines Entnahmeports 4. In den Hohlraum 2 des Entnahmeports 4 mündet ein Kanal 5 von der Gehäuseaußenseite her und ein gehäuseinterner Kanal 6, der die Hohlräume 2 und 3 verbindet. Eine Längsachse 7 des Kanals 5 ist in Einstechrichtung 8 einer Spritze (vgl. Fig. 2) zu einer Längsachse 9 des Kanals 6 versetzt; dabei ist der Kanal 5 einer Oberseite 10 des Gehäuses 1 zugewandt.

Zur Oberseite 10 des Gehäuses 1 hin ist der Hohlraum 2 durch einen Verschlußstopfen 11, der beispielsweise aus einem thermoplastischem Elastomerwerkstoff hergestellt ist, verschlossen. Der in Draufsicht kreisförmige Verschlußstopfen 11 ist in einer Nut 12, einer zylindrischen Öffnung 13 des Hohlraumes 2 eingeklemmt und dichtet somit den Hohlraum 2 gegenüber der das Gehäuse 1 umgebenden Atmosphäre ab.

In den zweiten Hohlraum 3 des Gehäuses 1 ist ein Küken 14 eines Hahns 15 eingesetzt. Das Küken 14 ist um eine Längsachse 16 relativ zum Gehäuse 1 drehbar. Koaxial zur Drehachse 16 des Kükens 14 sind im Küken buchsenartige Ausnehmungen 17 bzw. 18 vorgesehen, zwischen denen ein senkrecht zur Drehachse 16 verlaufender Kanal 19 vorgesehen ist, der das Küken mittig durchsetzt. In der gezeigten Drehstellung des Kükens ist der Kanal 19 des Kükens 14 koaxial zum Kanal 6 des Gehäuses 1 ausgerichtet. Durch den Kanal 19 gebildete Öffnungen 20 bzw. 21 sind zur Oberseite 10 des Gehäuses 1 hin durch eine das Küken 14 umschließende ringförmige Dichtung 23 umschlossen und zur Unterseite des Gehäuses 1 hin durch eine entsprechende Dichtung 22. Beide Dichtungen 22, 23 sind teilweise in das Küken eingelassen und liegen eng an dem zweiten Hohlraum 3 an; dadurch dichten sie den Kanal 19 des Kükens gegenüber dem Gehäuse 1 ab.

Zur Arretierung des Kükens in Richtung seiner Drehachse 16 ist im unteren Bereich des Kükens 14 eine konische Rastkerbe 24 und im oberen Bereich des Kükens 14 ein Absatz 25 vorgesehen. Der Absatz 25 stützt das Küken gegenüber der Oberseite 10 des Gehäuses 1 ab und die konische Rastkerbe 24 ist in einen entsprechenden Absatz 26 des Gehäuses eingerastet, so daß das Küken 14 axial fixiert aber drehbar ist.

Zum Drehen des Kükens 14 ist im oberen Bereich des Kükens 14 ein hebelartiger Griff 26 vorgesehen. In der gezeigten, geöffneten Stellung des Hahns 15 überdeckt der Griff 26 die zylindrische Öffnung 13 des Hohlraumes 2 und blockiert dadurch den Zugang zum Hohlraum 2. Der Winkelbereich, in dem das Küken 14 um die Drehachse 16 drehbar ist, ist festgelegt und zwar durch Drehanschläge 27 und 28, die Teil des Gehäuses 1 sind und durch zwei Drehanschläge (vgl. Fig. 2), die Teil des Kükens sind, von denen aber in der gezeigten Stellung des Hahns 15 nur der Drehanschlag 29 zu sehen ist. In der gezeigten Stellung liegt der Drehanschlag 29 am Drehanschlag 28 an.

Der Kanal 5 des Gehäuses 1, der in den Hohlraum 2 mündet, ist in Form eines Anschlußstutzens 30 etwas vom Gehäuse 1 weggeführt. Auf den Anschlußstutzen 30 ist ein Schlauch 31 aufgeschoben. Durch eine wulstartige Erhebung 32 an der Außenseite des Anschlußstutzens 30 wird der aufgeschobene Schlauch 31 etwas aufgeweitet und ist somit durch die zwischen dem Schlauch 31 und dem Anschlußstutzen 30 entstehende Reibkraft fixiert. Alternativ dazu kann der Schlauch 31 auch über eine Luer-Verbindung mit dem Anschlußstutzen gekoppelt sein.

Ein weiterer Anschlußkanal 33 des Gehäuses 1 mündet in den zweiten Hohlraum 3, liegt koaxial zum Kanal 6 und in der gezeigten Stellung des Hahns 15 auch koaxial zum Kanal 19. Über einen dem Anschlußstutzen 30 entsprechenden Anschlußstutzen 34 ist der Kanal 33 nach außen geführt. Auf den Anschlußstutzen 34 ist ein Schlauch 35 aufgeschoben und über eine wulstartige Erhebung 36 fixiert.

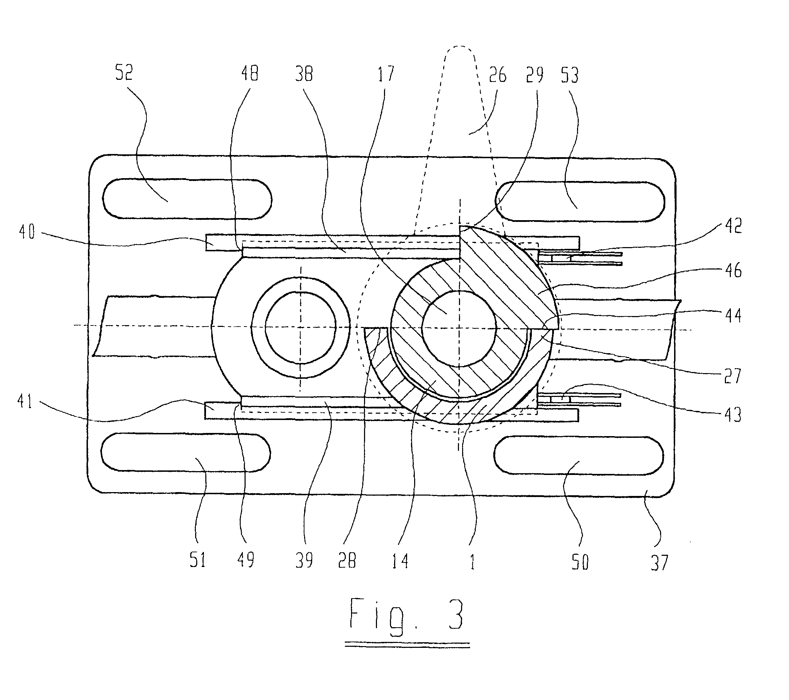

Das Gehäuse 1 ist auf einer ebenen Grundplatte 37 befestigt. Diese Grundplatte kann auch einstückig mit dem Gehäuse integriert sein und in einem Arbeitsgang durch Spritzgießen zusammen mit dem Gehäuse hergestellt werden. Zur Befestigung des Gehäuses 1 weist das Gehäuse 1 seitliche Befestigungsblätter 38 bzw. 39 (vgl. Fig. 3) auf, die in Halteschienen 40 bzw. 41 der Grundplatte 37 in Richtung der Längsachse 9 eingeschoben sind. Das Herausziehen des Gehäuses 1 aus den Halteschienen 41 und 42 wird durch Schnappelemente 42 bzw. 43 der Grundplatte 37, die am Gehäuse 1 anstehen, verhindert (vgl. Fig. 3); in den Fig. 1 und 2 ist nur das Schnappelement 42 zu sehen.

Für eine Blutentnahme ist der Anschlußstutzen 30 mit dem Patienten und der andere Anschlußstutzen 34 mit einem Reservoir verbunden. Eine nach der Blutabnahme vom Reservoir zum Patienten zurückgedrückte Spülflüssigkeit wird im Hohlraum 2 durch dessen Krümmung und durch den Versatz der beiden Kanäle 5 und 6 verwirbelt, wodurch Blutrückstände aus dem Entnahmeport 4 ausgespült werden.

Fig. 2 zeigt die Blutentnahmevorrichtung aus Fig. 1 in einer zweiten Stellung des Hahns 15. In dieser Stellung ist der Hahn 15 geschlossen, d.h. der in den Entnahmeport 4 mündende Kanal 6 ist durch das Küken 14 des Hahns 15 gegenüber dem Kanal 33 abgesperrt. Gegenüber der in Fig. 1 gezeigten Stellung des Hahns 15 ist das Küken 14 um 90° gedreht, so daß dessen hebelartiger Griff 26 in Fig. 2 hinter der Zeichenebene liegt und somit nicht sichtbar ist. Aufgrund der 90°-Drehung des Kükens 14 gegenüber dem Gehäuse 1 ist der mit dem Küken 14 verbundene Drehanschlag 29 (vgl. Fig. 1) ebenfalls nicht zu sehen. Stattdessen ist aber ein mit dem Küken 14 verbundener Drehanschlag 44 zu erkennen, der in der in Fig. 1 gezeigten Stellung des Hahns 15 durch das Küken 14 verdeckt ist. Der Drehanschlag 44 liegt in der in der Fig. 2 gezeigten Stellung des Hahns 15 am Drehanschlag 27 des Gehäuses 1 an. Somit ist der mögliche Drehwinkelbereich des Kükens 14 durch die mit dem Küken 14 verbundenen Drehanschläge 29 und 44 und die mit dem Gehäuse 1 verbundenen Drehanschläge 27 und 28 auf 90° begrenzt, weshalb ausschließlich eine Umschaltung von der in Fig. 1 gezeigten Öffnungsstellung des Hahns 15 in die in Fig. 2 gezeigte "Schließstellung" möglich ist.

In dieser Stellung des Hahns 15 ist die zylindrische Öffnung 13 des Entnahmeports 4 freigegeben und der Verschlußstopfen 11 kann mit einer Nadel 45 eines Blutentnahmegeräts 46 durchstochen werden. Somit kann mit dem Blutentnahmegerät 46 über die Nadel 45 aus dem Hohlraum 2 Blut abgenommen werden.

Das Blutentnahmegerät 46 ist vereinfacht als Spritze dargestellt. Selbstverständlich können alle gängigen Blutentnahmevorrichtungen hier verwendet werden. Je nach verwendetem Blutentnahmegerät kann die Öffnung 13 des Entnahmeports 4 auch so gestaltet sein, daß das Blutentnahmegerät formschlüssig am Entnahmeport angesetzt werden kann.

Fig. 3 zeigt einen Schnitt längs der Linie A-B der Fig. 2. Die Umrißlinie des Griffstücks 26 des Kükens 14 ist mit gestrichelter Linie eingezeichnet. Deutlich zu erkennen sind die Drehanschläge 29 und 44 des Kükens 14, die durch ein Ringsegment 46 mit einem Mittelpunktswinkel von 90° gebildet werden. Die Drehanschläge 27 und 28, die Teil des Gehäuses 1 sind, werden durch ein entsprechendes Ringsegment mit einem Mittelpunktswinkel von 180° des Gehäuses 1 gebildet.

Ferner ist zu erkennen, daß die Befestigungsblätter 38 und 39 des Gehäuses 1 mit etwa der Hälfte ihrer Breite und ihrer gesamten Länge in entsprechende Halteschienen 40 bzw. 41 der Grundplatte 37 eingeschoben sind. Ein Ende der Befestigungsblätter 38 und 39 steht an entsprechenden Absätzen 48 bzw. 49 der Halteschienen 40 bzw. 41 an. Die anderen Enden der Befestigungsblätter 38 bzw. 39 stehen an den am besten in den Fig. 1 und 2 zu erkennenden Schnappelementen 42 und 43 an. Die Schnappelemente 42 und 43 sind Teil der Grundplatte 37. Beim Einschieben der Befestigungsblätter 38 und 39 in die Halteschienen 40 und 41 werden die Schnappelemente 42 und 43 der Grundplatte 37 durch die Befestigungsblätter 38 und 39 nach unten, d.h. in entsprechende Ausnehmungen (nicht dargestellt) der Grundplatte 37 gedrückt. Ist das Gehäuse 1 so weit in die Halteschienen 40 und 41 eingeschoben, daß die Befestigungsblätter 38 und 39 an den entsprechenden Absätzen 48 bzw. 49 der Halteschienen 40 und 41 anstehen, so schnappen die Schnappelemente 42 und 43 nach oben. Somit ist das Gehäuse 1 zwischen den Schnappelementen 42 und 43 und den Absätzen 48 und 49 der Grundplatte 37 eingerastet und auf der Grundplatte 37 fixiert. Eine Demontage des Gehäuses 1 von der Grundplatte 37 ist möglich, indem die Schnappelemente 42 und 43 nach unten in die nicht dargestellten Ausnehmungen der Grundplatte 37 gedrückt werden und das Gehäuse 1 entgegen der Einschubrichtung aus den Halteschienen 40 und 41 gezogen wird.

Alternativ dazu ist es auch möglich, das Gehäuse 1 mit der Grundplatte 37 über Halteschienen 40 und 41 derart zu verbinden, daß die Fixierung ausschließlich über eine enge Passung zwischen den Befestigungsblättern 38 und 39 des Gehäuses 1 und den Halteschienen 40 und 41 erfolgt und keine Anschläge 48, 49 und Schnappelemente 42 und 43 vorgesehen sind.

In den Eckbereichen der Grundplatte 37 sind die Grundplatte 37 durchsetzende Langlöcher 50, 51, 52, 53 vorgesehen. Beispielsweise kann durch gegenüberliegende Langlöcher 50 und 53 bzw. 51 und 52 je ein Gurtband zur Befestigung der gesamten Blutentnahmevorrichtung am Arm oder Bein eines Patienten geführt werden.

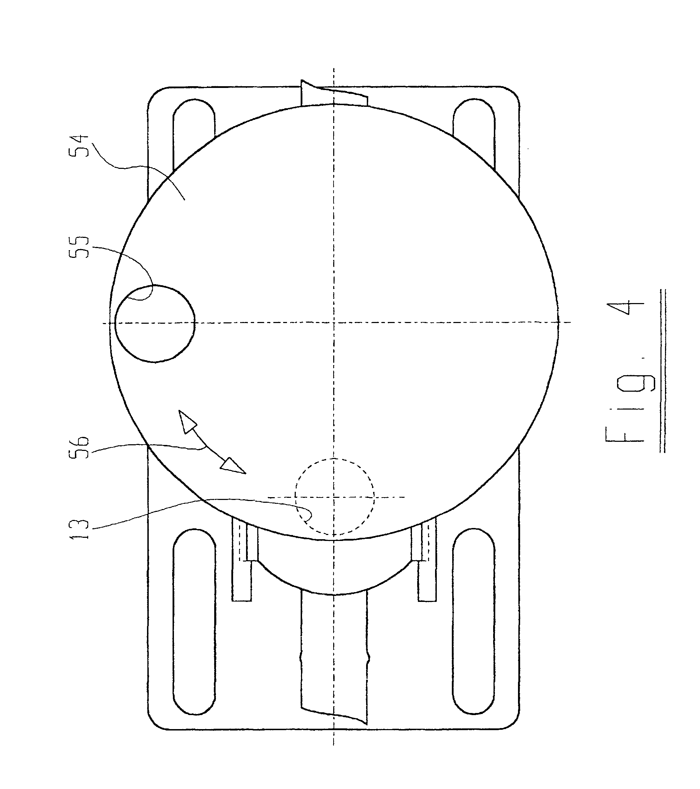

Fig. 4 zeigt ein weiteres Ausführungsbeispiel der Blutentnahmevorrichtung in Draufsicht. Dieses Ausführungsbeispiel entspricht im wesentlichen dem in den Fig. 1 bis 3 dargestellten Ausführungsbeispiel, unterscheidet sich jedoch dadurch, daß das Küken 14 einen kreisrunden, durch eine Platte gebildeten Griff 54, anstatt eines hebelartigen Griffs 26 (vgl. Fig. 1), aufweist. Dieser Griff 54 besitzt eine durchgehende kreisrunde Öffnung 55, deren Durchmesser im wesentlichen dem Durchmesser der zylindrischen Öffnung 13 des Entnahmeports 4 (vgl. Fig. 1) entspricht. Die zylindrische Öffnung 13 des Entnahmeports 4 wird in der gezeigten Stellung des Hahns 15 durch den Griff 54 verdeckt und ist deshalb mit gestrichelter Linie eingezeichnet. Durch eine 90°-Drehung des Griffs 54 kann die Öffnung 55 mit der zylindrischen Öffnung 13 des Entnahmeports 4 zur Deckung gebracht werden und eine Spritzennadel 45 kann in den Entnahmeport 4 eingeführt werden. Die Drehmöglichkeiten des Griffs 54 sind durch einen Doppelpfeil 56 angezeigt. Der mögliche Drehwinkelbereich des Griffs 54 ist durch Drehanschläge (in Fig. 4 nicht zu sehen), die den Drehanschlägen 27, 28, 29 und 44 der Fig. 1 und 2 entsprechen, begrenzt.

Alternativ zu der einen Öffnung 55 des Griffs 54 können auch mehrere Öffnungen im Griff 54 vorgesehen und der mögliche Drehwinkelbereich des Griffs 54 entsprechend erweitert sein.

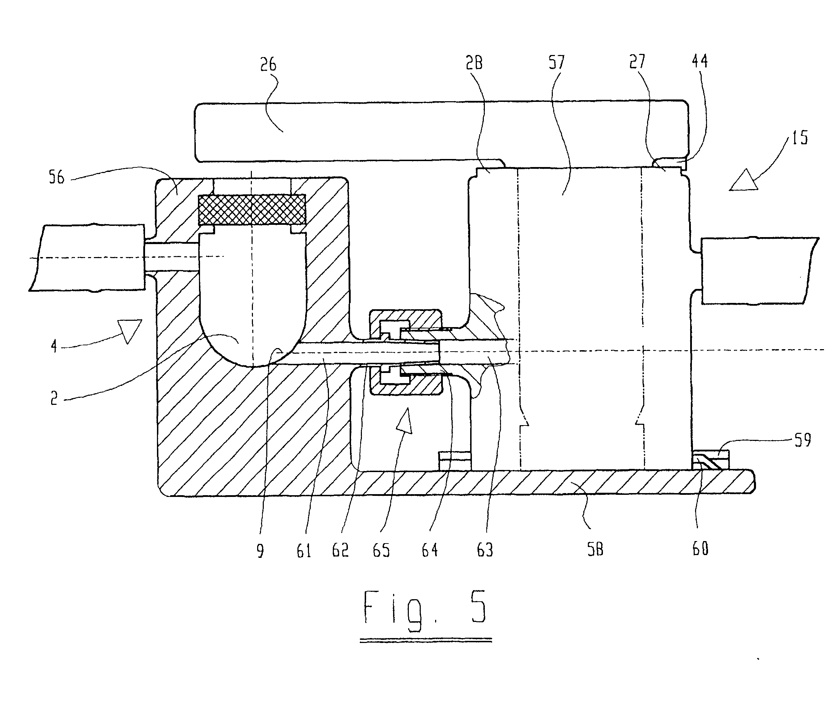

Fig. 5 zeigt ein weiteres Ausführungsbeispiel der Erfindung, bei dem der Entnahmeport 4 und der Hahn 15 als Bauteile mit separaten Gehäusen 56 bzw. 57 ausgeführt sind. Das Gehäuse 56 des Entnahmeports 4 erstreckt sich in seinem unteren Bereich auf der Seite des Kanals 6 in Form eines plattenartigen Abschnitts 58 vom Hohlraum 2 des Entnahmeports 4 weg. Ansonsten entspricht der Entnahmeport im wesentlichen dem Entnahmeport der Fig. 1 bis 4.

Auf dem plattenartigen Abschnitt 58 des Gehäuses 56 des Entnahmeports 4 ist das Gehäuse 57 des Hahns 15 befestigt. Die Befestigung des Hahns 15 auf dem plattenartigen Abschnitt 58 erfolgt analog zu den in den Fig. 1 bis 4 dargestellten Ausführungsbeispielen über Befestigungsblätter, Halteschienen 59, Anschläge und Schnappelemente 60.

Das Gehäuse 57 des Hahns 15 ist hier nicht geschnitten dargestellt und entspricht im wesentlichen der entsprechenden einen Hälfte des Gehäuses 1, in der der Hahn 15 angeordnet ist (vgl. Fig. 1 bis 3).

Ein im Gehäuse 56 angeordneter und in den Hohlraum 2 des Entnahmeports 4 mündender Kanal 61 ist in Form eines Anschlußstutzens 62 in den Zwischenraum zwischen dem Gehäuse 56 des Entnahmeports 4 und dem Gehäuse 57 des Hahns 15 nach außen geführt. Die beiden Anschlußstutzen 62 und 63 sind durch eine Luerverbindung 65 verbunden. In gleicher Weise ist ein Kanal 63 des Hahns 15 zu einem Anschlußstutzen 64 nach außen geführt.

Alternativ zu dem in Fig. 5 gezeigten Ausführungsbeispiel ist es auch möglich, eine dem Abschnitt 58 entsprechende Platte am Gehäuse 57 des Hahns 15 vorzusehen, auf der dann das Gehäuse 56 des Entnahmeports 4 befestigt wird, oder den Hahn 15 und den Entnahmeport 4 an einer separaten Halteplatte zu befestigen. Ferner ist es möglich, das Gehäuse 56 des Entnahmeports 4 mit dem Gehäuse 57 des Hahns 15 durch vielfältige andere Verbindungsmittel, wie z.B. Schrauben, Nieten oder durch Schweißen (z.B. Ultraschallschweißen) etc. miteinander zu verbinden oder auch nur durch die Luer-Verbindung 65.

## Patentansprüche

1. Blutentnahmevorrichtung mit einem Entnahmeport und einem Hahn, wobei der Entnahmeport einen Hohlraum aufweist, in den zwei Kanäle und eine Öffnung, die durch einen mit einer Nadel durchdringbaren Verschluß verschlossen ist, münden und der Hahn mit einem der Kanäle verbunden ist, **dadurch gekennzeichnet, daß** der Entnahmeport (4) und der Hahn (15) zueinander raumfest angeordnet sind und ein bewegliches Teil des Hahns (15) den Verschluß (11) in mindestens einer Stellung des Hahns (15) überdeckt und in mindestens einer anderen Stellung freigibt.

2. Blutentnahmevorrichtung nach Anspruch 1, **dadurch gekennzeichnet,**
**daß** das bewegliche Teil des Hahns (15), das den Verschluß (11) abdeckt oder freigibt, ein Griffstück (26, 54) des Hahns (15) ist.

3. Blutentnahmevorrichtung nach Anspruch 2, **dadurch gekennzeichnet,**
**daß** das Griffstück (26, 54) ein Betätigungshebel (26) ist.

4. Blutentnahmevorrichtung nach Anspruch 2, **dadurch gekennzeichnet,**
**daß** das Griffstück (26, 54) eine Platte (54) mit mindestens einer Öffnung (55) ist.

5. Blutentnahmevorrichtung nach einem der Ansprüche 1 bis 4, **dadurch gekennzeichnet,**
**daß** der Entnahmeport (4) und der Hahn (15) in ein gemeinsames Gehäuse (1) integriert sind.

6. Blutentnahmevorrichtung nach einem der Ansprüche 1 bis 5, **dadurch gekennzeichnet, daß** der Entnahmeport (4) und der Hahn (15) auf einer Grundplatte (37) befestigbar sind.

7. Blutentnahmevorrichtung nach Anspruch 6, **dadurch gekennzeichnet,**
**daß** der Entnahmeport (4) und der Hahn (15) mit einem Schnappverschluß (42, 43) auf der Grundplatte (37) befestigbar sind.

8. Blutentnahmevorrichtung nach Anspruch 6, **dadurch gekennzeichnet,**
**daß** der Entnahmeport (4) und der Hahn (15) über Halteschienen (40, 41) mit der Grundplatte (37) verbindbar sind.

9. Blutentnahmevorrichtung nach einem der Ansprüche 1 bis 4 oder 6 bis 8, **dadurch gekennzeichnet,**
**daß** der Entnahmeport (4) und der Hahn (15) unmittelbar und raumfest aneinander koppelbar sind.

10. Blutentnahmevorrichtung nach Anspruch 9, **dadurch gekennzeichnet,**
**daß** der Entnahmeport (4) und der Hahn (15) durch eine Luerverbindung (65) gekoppelt sind.

11. Blutentnahmevorrichtung nach einem der Ansprüche 6 bis 10, **dadurch gekennzeichnet,**
**daß** die Grundplatte (37) durch Gurte an einem Patienten befestigbar ist.

12. Blutentnahmevorrichtung nach einem der Ansprüche 1 bis 8, **dadurch gekennzeichnet,**
**daß** ein Hahnküken (14) des Hahns (15) in einem Winkelbereich von 90° relativ zu einem Gehäuse (1, 57) des Hahns (15) drehbar ist und ein Weiterdrehen des Hahnkükens (14) durch Drehanschläge (27, 28, 29, 44) blockiert ist.

## Claims

1. A blood sampling device comprising a sampling port and a tap, wherein the sampling port has a chamber, into which lead two ducts and an opening sealed by a stopper pierceable by a needle, and the tap is connected to one of the ducts, **characterised in that** the sampling port (4) and the tap (15) are arranged so as to be spatially fixed in relation to one another, and a movable part of the tap (15) covers the stopper (11) in at least one position of the tap (15) and uncovers it in at least one other position.

2. A blood sampling device according to claim 1, **characterised in that** the movable part of the tap (15), which covers or uncovers the stopper (11), is a gripping part (26, 54) of the tap (15).

3. A blood sampling device according to claim 2, **characterised in that** the gripping part (26, 54) is an operating lever (26).

4. A blood sampling device according to claim 2, **characterised in that** the gripping part (26, 54) is a plate (54) with at least one opening (55).

5. A blood sampling device according to any one of claims 1 to 4, **characterised in that** the sampling port (4) and the tap (15) are integrated into a common housing (1).

6. A blood sampling device according to any one of claims 1 to 5, **characterised in that** the sampling port (4) and the tap (15) are fixable to a base plate (37).

7. A blood sampling device according to claim 6, **characterised in that** the sampling port (4) and the tap (15) are fixable to the base plate (37) by means of a snap-locking connection (42, 43).

8. A blood sampling device according to claim 6, **characterised in that** the sampling port (4) and the tap (15) are connectable to the base plate (37) by means of mounting rails (40, 41).

9. A blood sampling device according to any one of claims 1 to 4 or 6 to 8, **characterised in that** the sampling port (4) and the tap (15) are directly connectable to one another in a spatially fixed manner.

10. A blood sampling device according to claim 9, **characterised in that** the sampling port (4) and the tap (15) are connected by means of a Luer connection (65).

11. A blood sampling device according to any one of claims 6 to 10, **characterised in that** the base plate (37) is fixable to a patient by means of belts.

12. A blood sampling device according to any one of claims 1 to 8, **characterised in that** a plug (14) of the tap (15) is rotatable within an angular range of 90° relative to a housing (1, 57) of the tap (15), and further rotation of the plug (14) is blocked by rotation stops (27, 28, 29, 44).

## Revendications

1. Dispositif de prélèvement d'échantillons de sang, qui comprend un port de prélèvement et un robinet, dans lequel le port de prélèvement présente une cavité dans laquelle débouchent deux canaux et une ouverture fermée par un bouchon pénétrable avec une aiguille, et le robinet est relié à l'un des canaux, **caractérisé en ce que**
le port de prélèvement (4) et le robinet (15) sont disposés de telle sorte qu'ils sont fixes dans l'espace l'un par rapport à l'autre, et une partie mobile du robinet (15) recouvre le bouchon (11) dans au moins une position du robinet (15) et le libère dans au moins une autre position.

2. Dispositif de prélèvement d'échantillons de sang selon la revendication 1, **caractérisé en ce que**
la partie mobile du robinet (15) qui couvre ou qui libère le bouchon (11) est un élément de préhension (26, 54) du robinet (15).

3. Dispositif de prélèvement d'échantillons de sang selon la revendication 2, **caractérisé en ce que**
l'élément de préhension (26, 54) est un levier de manoeuvre (26).

4. Dispositif de prélèvement d'échantillons de sang selon la revendication 2, **caractérisé en ce que**
l'élément de préhension (26, 54) est une plaque (54) présentant au moins une ouverture (55).

5. Dispositif de prélèvement d'échantillons de sang selon l'une des revendications 1 à 4, **caractérisé en ce que**
le port de prélèvement (4) et le robinet (15) sont intégrés dans un corps commun (1).

6. Dispositif de prélèvement d'échantillons de sang selon l'une des revendications 1 à 5, **caractérisé en ce que**
le port de prélèvement (4) et le robinet (15) peuvent être fixés sur une plaque de base (37).

7. Dispositif de prélèvement d'échantillons de sang selon la revendication 6, **caractérisé en ce que**
le port de prélèvement (4) et le robinet (15) peuvent être fixés sur la plaque de base (37) avec une fermeture à déclic (42, 43).

8. Dispositif de prélèvement d'échantillons de sang selon la revendication 6, **caractérisé en ce que**
le port de prélèvement (4) et le robinet (15) peuvent être reliés à la plaque de base (37) par des rails de maintien (40, 41).

9. Dispositif de prélèvement d'échantillons de sang selon l'une des revendications 1 à 4 ou 6 à 8, **caractérisé en ce que**
le port de prélèvement (4) et le robinet (15) peuvent être couplés ensemble directement et en étant fixes dans l'espace.

10. Dispositif de prélèvement d'échantillons de sang selon la revendication 9, **caractérisé en ce que**
le port de prélèvement (4) et le robinet (15) sont couplés par un raccord Luer (65).

11. Dispositif de prélèvement d'échantillons de sang selon l'une des revendications 6 à 10, **caractérisé en ce que**
la plaque de base (37) peut être fixée à un patient par des sangles.

12. Dispositif de prélèvement d'échantillons de sang selon l'une des revendications 1 à 8, **caractérisé en ce que**
un boisseau (14) du robinet (15) peut tourner dans une plage angulaire de 90° par rapport à un corps (1, 57) du robinet (15), et des butées de rotation (27, 28, 29, 44) empêchent le boisseau (14) de continuer à tourner.
